# EUROPEAN PATENT SPECIFICATION

(11) **EP 0 515 811 B1**
(45) Date of publication and mention of the grant of the patent: **02.08.2000**
(21) Application number: 92106280.8
(22) Date of filing: 10.04.1992
(51) Int. Cl.: B29C 65/20, A61M 39/00, B29L 23/22

(54) **Sterile welding of plastic tubes**
Steriles Schweissen von Kunststoffschläuchen
Soudage stérile de tuyaux en matière plastique

(30) Priority: 31.05.1991 US 708198
(43) Date of publication of application: 02.12.1992
(73) Proprietor: DENCO, INC., Wilmington, DE 19809 (US)
(72) Inventor: Shaposka, John B., Wilmington, DE 19808 (US); Spencer, Dudley W., Wilmington, DE 19803 (US)
(74) Representative: Wagner, Karl H., Dipl.-Ing.

(56) References cited:
- EP-A- 0 044 204
- EP-A- 0 471 953
- CH-A- 177 987
- US-A- 1 719 206
- US-A- 2 389 560
- US-A- 2 647 555
- US-A- 3 142 606
- US-A- 3 994 767
- US-A- 4 753 697

## Description

### Background of Invention

The present invention relates to the sterile and total containment welding techniques for plastic tubes such as fluid filled tubes. There are many applications which require a plastic tube to be connected to another plastic tube. Such applications include, for example, the taking of samples from a bioreactor, the treating of blood, CAPD and other medical applications. Prior patents generally dealing with this subject include U.S. Patents 4,793,880; 4,610,670; 4,369,779; 4,753,697; 4,770,735; 4,897,773; 4,897,138; 4,913,756; and 4,864,101.

It has been found that in the practice of U.S. Patent 4,610,670 an unacceptable level of PVC particles results when the cold seal is reopened after making a weld. The key features in the practice of the '670 patent involve cutting through the flattened areas of the tube and reopening the cold weld. Great care must be taken in using this technology because any residual liquid adversely lowers the hot wafer temperature which was already borderline. When welding through liquid tubes the clamped spacing must be less than one milliliter to be truly flat and expel all of the liquid. As the spacing increases the residual liquid pool gets larger and as a result, the tubes are not touching. When the clamp spacing reaches two millimiters the tube walls in the residual liquid area also are open about two millimiters.

### Summary of Invention

An object of this invention is to provide a device and a process for forming sterile connections with liquid filled tubes which overcomes the above disadvantages.

The above object is achieved by a method and a device as set forth in claims 1 and 10, respectively. Preferred embodiments of the invention are claimed in the dependent claims.

In one practice of this invention a pair of plastic tubes is clamped in a clamping device for each tube wherein each of the tubes is held in a bent condition in two sections. Each of the sections has an internal bubble with the bubbles being aligned. A cut is made through the aligned bubbles and the section of one tube is thereafter welded to a section of the other tube.

In the above practice, the invention may utilize a device wherein the bent tubes are mounted parallel to each other and then shifted to align the two sections which are to be welded together. In a variation of that practice the bent tubes are initially aligned with each other and cuts are made through each of the tubes to result in the bent portions being removed from the welding area so that the exposed cut tube sections can be moved relatively toward each other for contact and welding.

### The Drawings

Figure 1 is a top plan view partly in section schematically showing a sterile welding system in accordance with one embodiment of this invention;
Figure 2 is a top plan view similar to Figure 1 showing the system in a later stage of operation;
Figure 3 is a top plan view of a further embodiment of this invention in its initial stage of operation;
Figures 4-6 are plan views similar to Figure 3 showing later stages of operation;
Figure 7 is a graph plotting the bubble weight against the gap distance for use in the embodiments of Figures 1-6;

### Detailed Description

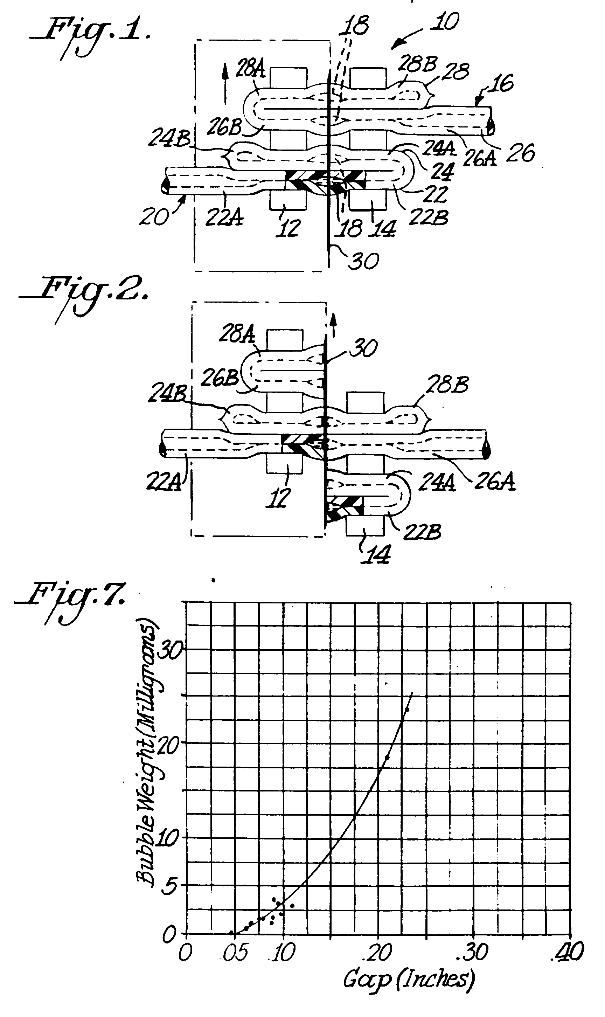

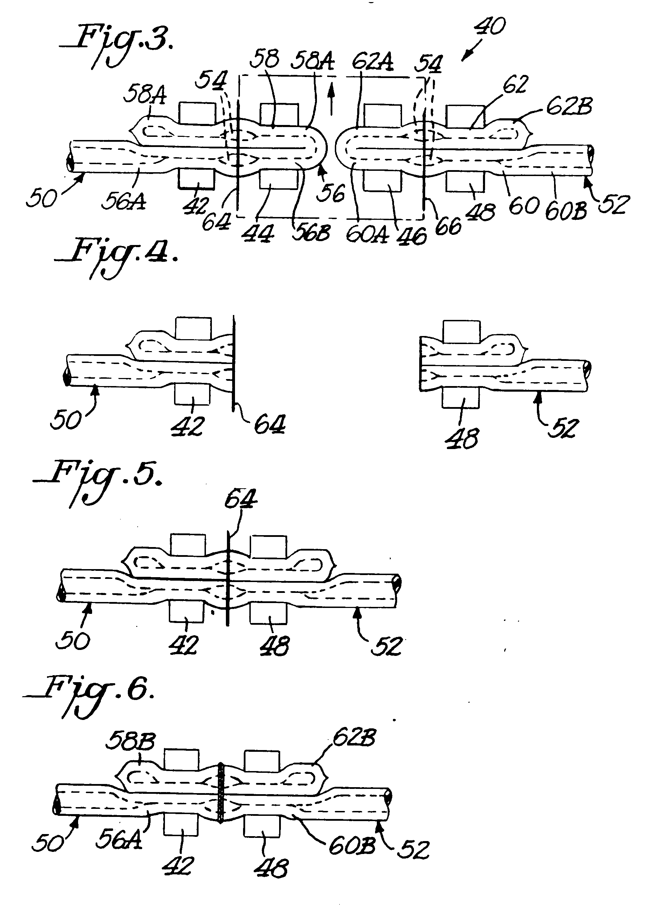

The present invention is directed to two different approaches at providing the sterile welding of plastic tubes. In the embodiments shown in Figures 1-6 the cutting of the tubes takes place through aligned bubbles with the sections to be welded together then being brought into contact with each other. In the embodiment of Figures 1-2 the tubes are mounted parallel to each other and the alignment of the sections to be welded together is effected by a shifting operation in a manner well known in the prior art. In the embodiment of Figures 3-6 the bent tubes are initially aligned with each other and the cut portions are removed from the welding area so that contact can be made by a transverse shifting of one or both cut tubes into contact with each other. It is to be understood that the invention can be practiced wherein the tube is cut by the conventional cut/shift method, such as in the '670 patent or by a melt/wipe process such as described in our copending applications, serial nos. 569,855 filed August 20, 1990; 604,979 filed October 29, 1990; and 682,977 filed April 10, 1991, the details of which are incorporated herein by reference thereto. For the sake of simplicity in terminology when reference is made to "cutting", the cutting can be of either type by either physically cutting through the tube or by a melt/wipe operation.

Figure 1 illustrates a sterile welding system 10 which includes a pair of holders 12,14 provided with known suitable clamping arrangements for clamping a pair of tubes 16,20. Each tube is shown as extending across both holders and bent to form two sections 22,24 and 26,28.

As also shown in Figure 1, each section of the bent tubes 16,20 includes a bubble 18. Because the clamping takes place across each holder 12,14 the bubbles 18 result in the unclamped areas between the holders. Thus, all of the bubbles are aligned with each other in the gap between the holders.

As illustrated in Figure 1 a suitable cutting device such as a heated wafer 30 is mounted for movement in the gap between holders 12,14 to cut through the bent tubes with the cutting action taking place through the bubble area. Where the tube is fluid filled, the bubbles are likewise fluid filled otherwise the bubbles would be dry or air filled. The cutting through aligned bubbles differs from our U.S. Patent 4,832,773 where the cutting is through a single air pocket.

After heated wafer 30 has completely cut through both bent tubes 16,20 one of the holding devices such as holding device 12 is shifted in a known manner, as indicated by the arrow, so that tube section 22A becomes aligned with tube section 26A and tube section 24B becomes aligned with tube section 28B. Two stub sections are formed which are not aligned with any other tube sections. These two stub sections comprise the bent portion 22B,24A and the bent portion 26B,28A. All of the various cut sections remain in contact with the heated wafer in the sequence of operation shown in Figure 2. Accordingly, the stub ends become sealed when the wafer 30 is removed whereas the aligned tube sections are disposed toward each other and then move into contact with each other and welded together by a transverse shifting of one holder toward the other in a manner known in the prior art.

Figures 3-6 illustrate a variation of the invention which involves the cutting through the bubble sections of tubes without requiring a shifting of the holders. As shown in Figure 3, device 40 includes a first pair of holders 42,44, each of which has their clamping member and a second pair of holders 46,48 which likewise has its clamping devices for clamping each bent tube 50,52 at two spaced locations thereby creating a series of bubbles 54 at each portion between the respective pairs of holders. Tube 50 is bent to form two portions 56,58 while tube 52 is bent to form two sections 60,62. Each portion in turn includes a section on each side of the bubble designated by the letters A and B.

Device 40 differs from device 10 in that the tubes 50,52 are initially aligned with device 40 whereas the corresponding tubes are parallel to each other with device 10. A wafer 64,66 is disposed for cutting through each bent tube through the bubble section. If desired the wafers 64,66 may comprise separate wafers which move simultaneously or sequentially. Alternatively, the same wafer may move sequentially through each bent tube to effect a cut through the bubble portions. After the cuts have been made the bent portions of tube 50, namely portions 56B and 58A and the bent portions 60A and 62A of tube 52 and their respective holders 44,46 are moved away from the welding area as shown in Figure 4. Where a heated wafer is used the resulting stubs are sealed and may be discarded.

After the stubs and their holders have been removed from the welding area it is necessary that the exposed ends of the remaining tube sections be heated for welding purposes. Where wafers 64,66 are heated the residual heat may be sufficient for these purposes. Otherwise, a heater such as heated wafer 64 may again be brought into contact with the exposed tube sections. This arrangement particularly lends itself to use of the melt/wipe techniques described in our aforenoted patent applications.

Figure 5 illustrates a practice of the invention where the heated wafer 64 is disposed against the exposed ends of cut tube 50. Holder 48 is then laterally shifted so that the exposed ends or faces of tube 52 are also brought into contact with heated wafer 64 as illustrated in Figure 5. Heated wafer 64 is removed after the exposed surfaces have been sufficiently heated and the exposed surfaces are then brought into contact with each other so that a closed tube is created from sections 56A and 60B with a closed stub being created from sections 58B and 62B, as shown in Figure 6.

Figure 7 illustrates the relationship between the bubble weight of the individual bubbles 18 or 54 and the gap distance between the adjacent clamping devices such as between holders 12 and 14 or holders 42 and 44 and holders 46 and 48.

The invention as practiced with systems 10 and 40 overcomes the disadvantages resulting from prior art such as indicated with respect to U.S. Patent 4,610,670. In the practices of the invention the tubes are not flat and the cooled weld is self-opening. Welding a sterile connection with a controlled liquid pool has a number of advantages. For example, such welding could be used to avoid particulates. Thus, the disadvantage of the prior art would be avoided wherein upon breaking open of the seal particles might be sent into the patient. A further advantage of the invention is that the tubes are self-opening after the weld is made. With a blade or wafer at 750°F and above use can be made of the "Ledenfrost Effect" to create a positive vapor pressure at the weld site to prevent incursion of bacteria. Because of the "Ledenfrost" seal protection a sterile weld can be made without a continuous melt pool as taught by prior art patents 4,369,779 and 4,610,670. Moreover, the technology is applicable to both cut/shift and melt/wipe processes.

Device 10 achieves a weld by handling four tube sections without shifting blocks or urging the tubes together although the urging aspect is a non-preferred option.

While the preferred practice is to cut by a heated wafer, the cutting and heating steps may be sequentially performed. The heater for the wafer may be battery operated, or may be electrically operated.

It is to be understood that various aspects described with respect to specific embodiments may be used with other embodiments.

## Claims

1. A method for the sterile welding of plastic tube sections comprising the steps of bending a pair of tubes (16, 20) with a section of each tube bent toward an adjacent section of the same tube to create four sections from the two tubes (16, 20), mounting each bent tube in a clamped condition across a pair of adjacent but spaced tube holders (12, 14) to create a bubble (18) in each tube section in the gap between the tube holders (12, 14), cutting through the tube sections at the bubbles (18) by relatively moving a cutting device (30) through the gap, aligning a tube section of one tube (16, 20) with a tube section of the other tube (20, 16), heating the cut ends of the aligned tube sections, and pressing the aligned ends together to weld the aligned tube sections.

2. The method of claim 1 wherein said steps of cutting and heating the tube sections are done simultaneously with a heated wafer (30).

3. The method of claim 1 or 2 wherein the tubes (16, 20) are arranged parallel to each other to provide four aligned bubbles (18), after the tube sections are cut the one tube section is aligned with the other tube section by a longitudinal shifting of the holders (12, 14) relative to each other, and two stub ends being created from the bent sections with two further stub ends welded to each other as well as the aligned tube sections being welded to each other.

4. The method of anyone of claims 1 to 3 wherein the tubes (16, 20) are fluid filled.

5. The method of any of the preceding claims, wherein the bent tubes are mounted to each other by each bent tube being mounted across a separate pair of tube holders, the tubes being cut by a cutting device cutting through pairs of aligned bubbles in each tube, after the two sections are cut the bent portions are removed away from the remaining tube sections, and the remaining tube sections are shifted relatively toward each other to contact the remaining tube sections of one tube with the tube sections of the other tube.

6. The method of any of the preceding claims, wherein the cutting through the two sets of bubbles is done simultaneously by two cutting devices.

7. The method of anyone of claims 1 to 5, wherein the cutting through the two sets of bubbles is done sequentially by the same cutting device.

8. The method of claim 7, wherein the cutting device remains in contact with one of the sets of tube sections, heating the cutting device, and the other set of tube sections being brought into contact with the heated cutting device to simultaneously heat all four exposed tube sections.

9. The method of claim 8, wherein two stub ends are welded to each other simultaneously with the aligned tube sections being welded to each other.

10. A device (10, 40) for the sterile welding of plastic tube sections, comprising adjacent but spaced tube holders (12, 14); 42, 44; 46, 48) for receiving a pair of tubes (16, 20) with a section of each tube being bent toward an adjacent section of the same tube to create four sections from the two tubes (16, 20) and for mounting each bend tube in a clamped condition across said tube holders (12, 14; 42, 44; 46, 48) such that a bubble (18; 54) is created in each tube section in a gap between the tube holders (12, 14; 42, 44; 46, 48);
cutting means for cutting through the tube sections at the bubbles (18; 54) by relatively moving a cutting device (30; 64, 66) through said gap;
alignment means for aligning a tube section of one tube (16, 20; 50, 52) with a tube section of the other tube; heating means for heating the cut ends of the aligned tube sections; and
means for moving and pressing the aligned ends together to weld the aligned tube section to each other.

11. The device of claim 10, wherein said cutting means and said heating means are provided by a heated waver (30; 64, 66).

12. The device of claim 10 or 11, wherein said tube holders (12, 14) are arranged parallel to each other and wherein the alignment means provide a longitudinal shifting of the holders (12, 14) relative to each other.

13. The device of claim 10 or 11, wherein said tube holders (42, 44; 46, 48) are substantially arranged on one line such that the tubes (50; 52) are initially aligned, and wherein means for removing the two center tube holders (44, 46) and for moving the remaining tube holders (42, 48) toward each other are provided.

14. The device (40) of claim 13, wherein two cutting means (64, 66) are provided for cutting through the bubbles (54).

## Patentansprüche

1. Verfahren zum sterilen Schweißen von Plastikschlauchabschnitten, wobei die folgenden Schritte vorgesehen sind:
Biegen eines Paars von Schläuchen (16, 20), wobei ein Abschnitt jedes Schlauches zu einem benachbarten Abschnitt des gleichen Schlauches gebogen wird, um vier Abschnitte aus den zwei Schläuchen (16, 20) zu bilden,
Anbringen jedes gebogenen Schlauches in einem geklemmten Zustand über einem Paar von benachbarten aber beabstandeten Schlauchhaltern (12, 14) zur Erzeugung einer Blase (18) in jedem Schlauchabschnitt im Spalt zwischen den Schlauchhaltern (12, 14),
Schneiden der Schlauchabschnitte an den Blasen (18) durch Relativbewegung einer Schneidvorrichtung (30) durch den Spalt,
Ausrichten eines Schlauchabschnittes eines Schlauches (16, 20) mit einem Schlauchabschnitt des anderen Schlauches (20, 16),
Erhitzen der abgeschnittenen Enden der ausgerichteten Schlauchabschnitte, und
Pressen der ausgerichteten Enden zusammen zum Schweißen der ausgerichteten Schlauchabschnitte.

2. Verfahren nach Anspruch 1, wobei die Schritte des Schneidens oder Erhitzens der Schlauchabschnitte gleichzeitig erfolgen, und zwar mit einer erhitzten Scheibe (30).

3. Verfahren nach Anspruch 1 oder 2, wobei die Schläuche (16, 20) parallel zueinander angeordnet sind, um vier ausgerichtete Blasen (18) vorzusehen, wobei nach dem Schneiden der Schlauchabscnitte, der eine Schlauchabschnitt mit dem anderen Schlauchabschnitt ausgerichtet ist, und zwar durch eine longitudinale Verschiebung der Halter (12, 14) relativ zueinander, und wobei zwei Stummelenden erzeugt werden aus den gebogenen Abschnitten mit zwei weiteren Stummelenden verschweißt miteinander und auch mit den ausgerichteten Schlauchabschnitten die miteinander verschweißt werden.

4. Verfahren nach einem der Ansprüche 1 bis 3, wobei die Schläuche (16, 20) mit einem Strömungsmittel gefüllt sind.

5. Verfahren nach einem der vorhergehenden Ansprüche, wobei die gebogenen Schläuche aneinander angebracht sind, und zwar dadurch daß jeder gebogene Schlauch über einem gesonderten Paar von Schlauchhaltern angebracht ist, wobei die Schläuche durch eine Schneidvorrichtung geschnitten werden, welche durch Paare von ausgerichteten Blasen in jedem Schlauch schneidet, wobei nach dem Schneiden der zwei Abschnitte die gebogenen Teile von den verbleibenden Schlauchabschnitten entfernt, und wobei ferner die verbleibenden Schlauchabschnitte relativ zueinander verschoben werden, um die verbleibenden Schlauchabschnitte des einen Schlauches mit den Schlauchabschnitten des anderen Schlauches zu kontaktieren.

6. Verfahren nach einem der vorhergehenden Ansprüche, wobei das Schneiden durch die zwei Sätze von Blasen gleichzeitig durch zwei Schneidvorrichtungen erfolgt.

7. Verfahren nach einem der Ansprüche 1 bis 5, wobei das Schneiden durch die zwei Sätze von Blasen sequentiell durch die gleiche Schneidvorrichtung erfolgt.

8. Verfahren nach Anspruch 7, wobei die Schneidvorrichtung in Kontakt mit einem der Sätze von Schlauchabschnitten verbleibt, die Schneidvorrichtung erhitzt und der andere Satz von Schlauchabschnitten in Kontakt gebracht wird mit der erhitzten Schneidvorrichtung, um gleichzeitig alle vier freiliegenden Schlauchabschnitte zu erhitzen.

9. Verfahren nach Anspruch 8, wobei die zwei Stummelenden miteinander verschweißt werden, gleichzeitig mit dem Zusammenschweißen der ausgerichteten Rohrabschnitte.

10. Vorrichtung (10, 40) zum sterilen Schweißen von Plastikschlauchabschnitten, wobei folgendes vorgesehen ist:
benachbarte aber beabstandete Schlauchhalter (12, 14; 42, 44; 46, 48) zur Aufnahme des Paars von Schläuchen (16, 20) mit einem Abschnitt jedes Schlauches gebogen zu einem benachbarten Abschnitt des gleichen Schlauches hin, um vier Abschnitte aus den zwei Schläuchen (16, 20) zu bilden, und um jeden gebogenen Schlauch in einem geklemmten Zustand über die Schlauchhalter (12, 14; 42, 44; 46, 48) derart anzuordnen, daß eine Blase (18; 54) in jedem Schlauchabschnitt in einem Spalt zwischen den Schlauchhaltern (12, 14; 42, 44; 46, 48) gebildet wird;
Schneidmittel zum Schneiden durch die Schlauchabschnitte an den Blasen (18; 54) durch Relativbewegung einer Schneidvorrichtung (30; 64,66) durch den Spalt;
Ausrichtmittel zum Ausrichten eines Schlauchabschnittes eines Schlauches (16, 20; 50, 52) mit einem Schlauchabschnitt des anderen Schlauches; Erhitzungsmittel zum Erhitzen der abgeschnittenen Enden der ausgerichteten Schlauchabschnitte; und
Mittel zur Bewegung und zum Pressen der ausgerichteten Enden zusammen zum Schweißen des ausgerichteten Schlauchabschnitts miteinander.

11. Vorrichtung nach Anspruch 10, wobei die Schneidmittel und die Heizmittel durch eine beheizte Scheibe (30; 64, 66) vorgesehen sind.

12. Vorrichtung nach Anspruch 10 oder 11, wobei die Schlauchhalter (12, 14) parallel zueinander angeordnet sind, und wobei die Ausrichtmittel eine Längsverschiebung der Halter (12, 14) relativ zueinander vorsehen.

13. Vorrichtung nach Anspruch 10 oder 11, wobei die Schlauchhalter (42, 44; 46, 48) im wesentlich auf einer Linie derart angeordnet sind, daß die Schläuche (50; 52) anfangs ausgerichtet sind, und wobei Mittel zur Entfernung der zwei Mittelschlauchhalter (44, 46) und zur Bewegung der verbleibenden Schlauchhalter (42, 48) zueinander vorgesehen sind.

14. Vorrichtung (40) nach Anspruch 13, wobei zwei Schneidmittel (64, 66) zum Schneiden der Blasen (54) vorgesehen sind.

## Revendications

1. Procédé de soudage stérile de portions de tube plastique comprenant les étapes consistant à courber deux tubes (16, 20), une partie de chaque tube étant recourbée vers une partie voisine du même tube pour créer quatre portions à partir des deux tubes (16, 20), monter chaque tube recourbé dans un état bloqué en travers d'une paire de dispositifs de maintien de tube (12, 14) voisins mais espacés, pour créer une bulle (18) dans chaque portion de tube dans l'intervalle entre les dispositifs de maintien de tube (12, 14), couper à travers les portions de tube au niveau des bulles (18) en déplaçant de façon relative un dispositif de coupe (30) à travers l'intervalle, aligner une portion de tube d'un tube (16, 20) avec une portion de tube de l'autre tube (20, 16), chauffer les extrémités coupées des portions de tube alignées, et presser les extrémités alignées l'une sur l'autre pour souder les portions de tube alignées.

2. Procédé selon la revendication 1, dans lequel les étapes de coupe et de chauffage des portions de tube sont effectuées simultanément au moyen d'une lame chauffée (30).

3. Procédé selon la revendication 1 ou 2, dans lequel les tubes (16, 20) sont disposés parallèlement l'un à l'autre pour fournir quatre bulles alignées (18), après que les portions de tube sont découpées, la première portion de tube est alignée avec l'autre portion de tube par un décalage longitudinal des dispositifs de maintien (12, 14) l'un par rapport à l'autre, et deux extrémités tronquées sont créées à partir des portions recourbées, deux autres extrémités tronquées étant soudées l'une à l'autre de même que les portions de tube alignées sont soudées l'une à l'autre.

4. Procédé selon l'une quelconque des revendications 1 à 3, dans lequel les tubes (16, 20) sont remplis de fluide.

5. Procédé selon l'une quelconque des revendications précédentes, dans lequel les tubes recourbés sont montés l'un sur l'autre, chaque tube recourbé étant monté transversalement à une paire séparée de dispositifs de maintien de tube, les tubes étant coupés par un dispositif de coupe coupant à travers des paires de bulles alignées dans chaque tube, après que les deux portions sont coupées, les parties de tube sont enlevées des portions de tube restantes, et les portions de tube restantes sont décalées l'une vers l'autre pour contacter les portions de tube restantes d'un tube aux portions de tube de l'autre tube.

6. Procédé selon l'une quelconque des revendications précédentes dans lequel la coupe à travers deux ensembles de bulles est effectuée simultanément par deux dispositifs de coupe.

7. Procédé selon l'une quelconque des revendications 1 à 5, dans lequel la coupe à travers les deux ensembles de bulles est effectuée séquentiellement par le même dispositif de coupe.

8. Procédé selon la revendication 7, dans lequel le dispositif de coupe reste en contact avec l'un des ensembles de portions de tube, chauffant le dispositif de coupe, et l'autre ensemble de portions de tube étant amené en contact avec le dispositif de coupe chauffé pour chauffer simultanément les quatre portions de tube exposées.

9. Procédé selon la revendication 8, dans lequel les deux extrémités tronquées sont soudées l'une à l'autre simultanément avec la soudure des portions de tubes alignées.

10. Dispositif (10, 40) pour le soudage stérile de portions de tube plastique, comprenant des dispositifs de maintien de tube adjacents mais espacés (12, 14 ; 42, 44 ; 46, 48) pour recevoir deux tubes (16, 20), une portion de chaque tube étant recourbée vers une portion adjacente du même tube pour créer quatre portions à partir des deux tubes (16, 20) et pour monter chaque tube recourbé dans un état bloqué en travers des dispositifs de maintien de tube (12, 14 ; 42, 44 ; 46, 48) de sorte qu'une bulle (18 ; 54) est créée dans chaque portion de tube dans un intervalle entre les dispositifs de maintien de tube (12, 14 ; 42, 44 ; 46, 48) ; un moyen de coupe pour couper en travers des portions de tube au niveau des bulles (18 ; 54) en déplacant de façon relative un dispositif de coupe (30 ; 64, 66) en travers dudit intervalle ; un moyen d'alignement pour aligner une portion de tube d'un tube (16,20 ; 50, 52) avec une portion de tube de l'autre tube ; un moyen de chauffage pour chauffer des extrémités de coupe des portions de tube alignées ; et un moyen pour déplacer et presser l'une sur l'autre les extrémités alignées pour souder l'une à l'autre les portions de tube alignées.

11. Dispositif selon la revendication 10, dans lequel le moyen de coupe et le moyen de chauffage sont constitués d'une lame chauffée (30 ; 64, 66).

12. Dispositif selon la revendication 10 ou 11, dans lequel les dispositifs de maintien de tube (12, 14) sont disposés parallèlement et dans lequel le moyen d'alignement permet un décalage longitudinal des dispositifs de maintien (12, 14) l'un par rapport à l'autre.

13. Dispositif selon la revendication 10 ou 11, dans lequel les dispositifs de maintien de tube (42, 44 ; 46, 48) sont disposés sensiblement en alignement de sorte que les tubes (50 ; 52) sont initialement alignés, et dans lequel des moyens pour enlever les deux dispositifs de maintien de tube centraux (44, 46) et pour déplacer les dispositifs de maintien de tube restants (42, 48) l'un vers l'autre sont prévus.

14. Dispositif (40) selon la revendication 13, dans lequel deux moyens de coupe (64, 66) sont prévus pour couper en travers des bulles (54).
